# NEUE EUROPÄISCHE PATENTSCHRIFT

(11) **EP 0 912 508 B2**
(45) Veröffentlichungstag und Bekanntmachung des Hinweises auf die Entscheidung über den Einspruch: **07.12.2005**
(45) Hinweis auf die Patenterteilung: 16.01.2002
(21) Anmeldenummer: 97935513.8
(22) Anmeldetag: 07.07.1997
(51) Int. Cl.: C07D 201/08, C07D 201/12, C07D 201/16

(54) **VERFAHREN ZUR HERSTELLUNG VON CAPROLACTAM AUS 6-AMINOCAPRONITRIL**
PROCESS FOR PREPARING CAPROLACTAM FROM 6-AMINOCAPRONITRILE
PROCEDE DE PREPARATION DE CAPROLACTAME A PARTIR DE 6-AMINOCAPRONITRILE

(30) Priorität: 17.07.1996 DE 19628805
(43) Veröffentlichungstag der Anmeldung: 06.05.1999
(73) Patentinhaber: BASF Aktiengesellschaft, 67056 Ludwigshafen (DE)
(72) Erfinder: RITZ, Josef, D-67069 Ludwigshafen (DE); ACHHAMMER, Günther, D-68309 Mannheim (DE); FISCHER, Rolf, D-69121 Heidelberg (DE); FUCHS, Eberhard, D-67227 Frankenthal (DE); VOIT, Guido, D-69198 Schriesheim (DE)
(86) Internationale Anmeldenummer: PCT/EP1997/003569
(87) Internationale Veröffentlichungsnummer: WO 1998/003481

(56) Entgegenhaltungen:
- EP-A- 0 150 295
- EP-A- 0 209 021
- EP-A- 0 529 470
- EP-A- 0 659 741
- EP-A- 0 670 308
- EP-A- 0 748 797
- DE-A- 1 944 910
- DE-A- 4 319 134
- FR-A- 1 184 282
- FR-A- 2 029 540
- US-A- 2 301 964
- US-A- 2 357 484
- US-A- 5 495 016
- US-A- 5 496 941

## Beschreibung

Die vorliegende Anmeldung betrifft ein verbessertes Verfahren zur Herstellung von Caprolactam durch Cyclisierung von 6-Aminocapronitril in Gegenwart von Wasser bei erhöhter Temperatur und gewünschtenfalls eines Katalysators sowie eines Lösungsmittels.

Es ist bekannt, 6-Aminocapronitril mit Wasser zu Caprolactam und Ammoniak umzusetzen. Hierbei kann man sowohl in der Gasals auch in der Flüssigphase arbeiten. So erzielt man gemäß den US 4,628,085 und US 4,625,023 in der Gasphase in Gegenwart von Metalloxiden wie Aluminiumoxid, Siliziumdioxid oder gemäß der EP-A 659,741 in Gegenwart von Metallphosphaten hohe Caprolactam-Ausbeuten. Gemäß der US 2,301,964 gelingt auch in der Flüssigphase die Caprolactam-Herstellung mit hoher Ausbeute ohne Katalysator. Die Cyclisierung in der Flüssigphase in Gegenwart von Katalysatoren wie homogen gelösten Metallsalzen beschreibt die FR-A 2,029,540. Die DE-A 4,339,648 und die DE-A 4,422,610 beschreiben die Flüssigphasenreakfion in Gegenwart von suspendierten oder fest angeordneten Metalloxiden.

Unabhängig davon, wie man 6-Aminocapronitril zu Caprolactam cyclisiert, fallen üblicherweise neben dem gewünschten Wertprodukt Caprolactam als Nebenprodukte Hochsieder an, d.h. Verbindungen mit Siedepunkten, die höher als der Siedepunkt von Caprolactam liegen. Die Menge an diesen Hochsiedern kann je nach Art des gewünschtenfalls eingesetzten Cyclisierungskatalysators und den Bedingungen der Cyclisierung beträchtlich variieren.

So beschreibt US 5,495,016 ein Verfahren zur Herstellung von Caprolactam aus 6-Aminocapronitril, worin die Verringerung und Rückgewinnung der Hochsieder erzielt wird. Aus DE-A 19 44 910 ist bekannt, daß man durch Zugabe von Phosphorsäure und/oder Polyphosphorsäure die Umsetzung von 6-Aminocapronamid und 6-Aminocapronsäure in Caprolactam fördert.

Hauptbestandteile dieser Hochsieder sind Dimere und Oligomere unterschiedlicher Molekulargewichte. Die Zusammensetzung dieser Hochsieder unterscheidet sich von der Zusammensetzung der Polymeren, die man bei der Polymerisation von Caprolactam erhält.

Bei der Cyclisierung von 6-Aminocapronitril mit Wasser zu Caprolactam und Ammoniak entstehen Hochsieder der Formel I, 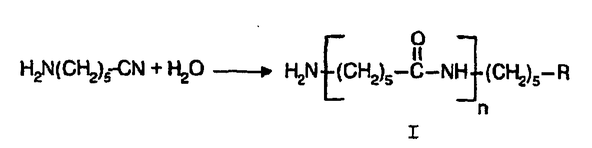 in der die Reste R Carbonsäure-, Carbonsäureamid-, und Nitrilgruppen und, bei Verwendung von Alkoholen als Lösungsmitteln bei der Cyclisierung von 6-Aminocapronitril, auch Estergruppen darstellen. Der Buchstabe n stellt dabei ganze Zahlen von 1 bis ca. 50 dar, wobei der Durchschnittswert von n über alle Verbindungen I im allgemeinen kleiner als 5 ist.

Demgsgenüber entstehen bei der ringöfinenden Polykondensation von Caprolactam in Gegenwart von Wasser, Polymerisate (Nylon 6) der Formel II:

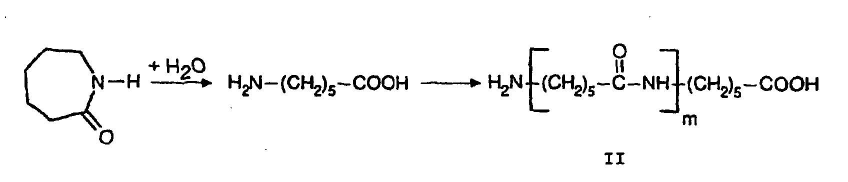

Im Gegensatz zu den Hochsiedern der Formel I liegen die Werte m bei den Polymeren der Formel II bei weit über Tausend und als Endgruppen R treten nur Carbonsäurereste auf.

Hieraus geht hervor, daß sich die Hochsiedergemische der Formel I in ihrer chemischen Struktur und ihren Molgewichten wesentlich von Nylon 6 unterscheiden, das aus Caprolactam gewonnen wird.

Es ist bekannt, Polyamid-6 oder Polyamid-6 enthattende Produktgemische zu Caprolactam zurückzuspalten. Das für die Synthese des Polyamids-6 verwendete Caprolactam wird bislang fast ohne Ausnahme durch Beckmannsche Umlagerung von Cyclohexanonoxim hergestellt. Die Spaltung des Polyamids-6 oder der Polyamid-6 enthaltenden Produktgemische erfolgt üblicherweise unter der Einwirkung saurer oder basischer Katalysatoren bei erhöhter Temperatur, häufig unter Einwirkung von Wasserdampf.

In Chem. Ing. Techn. 45 (1973) 1510 wird die technische Durchführung eines Spaltverfahrens mit überhitztern Wasserdampf beschrieben. ZurAufarbeitung ist dieAufkonzentrierung einerCaprolactam/Wasser-Lösung erforderlich.

Nach dem Verfahren der EP-A 209 021 wird die Spaltung in einem Aluminiumoxid-Wirbelbett unter Erhalt von Caprolactam durchgeführt. Die EP-A 529 470 lehrt den Zusatz von Kaliumcarbonat als Katalysator zur Polyamid-6-Spaltung, wobei die Reaktion bei 250 bis 320 °C unter gleichzeitigem Abdestillieren des Caprolactams im Vakuum durchgeführt wird.

In DE-A2,440,243 wird die Spaltung von Polyamid enthaltenden Textilabfällen mit Wasser und Phosphorsäure enthaltenden Säuregemischen wie Phosphorsäure und Phosphorige Säure, Phosphorsäure, Phosphorige Säure und Borsäure, Phosphorsäure und Salzsäure (Beispiele 1 bis 3) zu Caprolactam beschrieben.

Die DE-A 2,164,462 beschreibt ein Verfahren zur Herstellung von ε-Aminocapronsäure und cyclischem dimerem Caprolactam aus Oligomeren des Caprolactams, indem man die Oligomeren mit Schwefelsäure bei Temperaturen über 50°C behandelt, mit Wasser verdünnt, das dabei ausfallende cyclische dimere Caprolactam abtrennt, das Filtrat mit einem schwach basischen Ionenaustauscher behandelt, einengt und die auskristallisierende ε-Aminocapronsäure isoliert.

Die US 3,182,055 beschreibt ein kontinuierliches Verfahren zur Herstellung von Caprolactam durch Behandeln von Polycaprolactam mit 0,1 bis 5 Teilen Phosphorsäure pro 100 Teile des Polymers und Wasserdampf bei Temperaturen im Bereich von 220 bis 375°C und einem Druck im Bereich von 0,5 bis 6 bar, wobei das entstehende Caprolactam mit dem Wasserdampf aus dem System entfernt wird.

Aus WO 94/06763 ist ein kontinuierliches Verfahren zur Rückgewinnung von Caprolactam aus Teppichböden, die zum Teil aus Nylon 6 bestehen, bekannt. Die Spaltung des zerkleinerten, Nylon 6 enthaltenden Materials erfolgt durch Einleiten von überhitztem Wasserdampf in Gegenwart von Phosphorsäure. Aus den Beispielen 1, 3, 4 und 5 geht hervor, daß, bezogen auf den Nylon 6-Gehalt des Materials, 40 Gew.-%, 8 Gew.-%, 24 Gew.-% bzw. 60 Gew.-% Phosphorsäure benötigt werden. Die entsprechenden Caprolactam-Rohausbeuten betragen, ebenfalls bezogen auf den Nylon 6-Gehalt des Materials, 56 %, 37 %, 89 % bzw. 80 %.

Die bisherigen, vor allem kontinuierlich ablaufenden Verfahren zur Spaltung von Polyamid-6 oder Polyamid-6 enthaltendem Material zu Caprolactam weisen gravierende Nachteile auf. Einmal werden zur Abtrennung des Caprolactams relativ große Mengen Wasserdampf benötigt. Dieses Wasser muß später mit hohem Energieaufwand wieder von Caprolactam abgetrennt werden. Zum anderen entstehen aus Phosphorsäure und den nicht verwertbaren Hochsiederanteilen Destillationsrückstände, die entsorgt werden müssen. Diese Entsorgung kann in den meisten Fällen nur durch Deponieren erfolgen.

Aus dem zitierten Stand der Technik geht hervor, daß für eine mit hoher Caprolactam-Ausbeute verlaufende Rückspaltung von Polyamid-6 enthaltenden Polymeren je nach Zusammensetzung des Materials sehr unterschiedliche Mengen an Wasserdampf und Phosphorsäure benötigt werden.

Bei den Verbindungen der Formel I treten für den Rest R Carbonsäureamid- und Nitrilgruppen auf. Es ist bekannt, daß Carbonsäureamid- und Nitrilgruppen in Gegenwart von Säuren und Wasser Ammoniak bilden (siehe H. Beyer, Lehrbuch der organischen Chemie, Verlag S. Hirzel, Leipzig, 20. Auflage, S. 252)

Bei der Spaltung der Verbindungen I in Gegenwart einer geringen Menge an Säure ist daher zu erwarten, daß der gebildete Ammoniak zumindest einen Teil der zugesetzten Säure neutralisiert, so daß entweder mehr Säure zugegeben werden muß, oder die Spaltung nicht vollständig abläuft.

Aufgabe der vorliegenden Erfindung war es daher, ein mit möglichst hoher Caprolactam-Ausbeute und möglichst geringem Energie- und Säureaufwand durchführbares Verfahren zu entwickeln, mit dessen Hilfe die bei der 6-Aminocapronitril-Cyclisierung gebildeten Hochsieder in Caprolactam möglichst hoher Reinheit umgewandelt werden können. Des weiteren sollte ein Verfahren gefunden werden, bei dem eine Entsorgung durch Deponieren möglichst vollständig vermieden wird.

Demgemäß wurde ein verbessertes Verfahren zur Herstellung von Caprolactam und höher als Caprolactam siedenden Komponenten ("Hochsieder") der Formel I 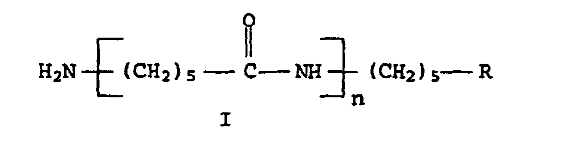 mit
- R:: Carbonsäure-, Carbonsäureamid-, und Nitrilgruppe und, bei Verwendung von Alkoholen als Lösungsmittel bei oder der Cyclisierung von 6-Aminocapronitril, zusätzlich und/oder Estergruppe
- n:: ganze Zahl von 1 bis 50, wobei der Durchschnittswert von n über alle Verbindungen I kleiner als 5 ist.
durch Cyclisierung von 6-Aminocapronitril in Gegenwart von Wasser bei erhöhter Temperatur und gewünschtenfalls eines Katalysators sowie eines Lösungsmittels gefunden, indem man
a) aus einem Reaktionsaustrag ("Reaktionsaustrag I") der Cyclisierung Caprolactam und Hochsieder abtrennt,
b) die Hochsieder aus Stufe a) mit 0,01 bis 10 Gew.-% Phosphorsäure und/oder Polyphosphorsäure, bezogen auf die eingesetzten Hochsieder, bei einer Temperatur im Bereich von 200 bis 350°C behandelt unter Erhalt eines Reaktionsaustrages II und
c) aus dem Reaktionsaustrag II der Stufe b) gebildetes Caprolactam und gegebenenfalls 6-Aminocapronitril von nicht umgesetzten Hochsiedern und eingesetzter Säure abtrennt.

Erfindungsgemäß setzt man aus 6-Aminocapronitril hergestelltes Caprolactam ein. Diese Cyclisierung von 6-Aminocapronitril kann man nach bekannten Verfahren in der Flüssig- oder Gasphase durchführen, beispielsweise nach einem Verfahren aus der US 2 301 964, US 2 357 484, EP-A 150 295 oder der DE-A 43 19 134, indem man üblicherweise das 6-Aminocapronitril mit Wasser in der Flüssigphase zu Caprolactam und Ammoniak umsetzt.

Bei der Umsetzung ohne Katalysator wählt man in der Regel eine Temperatur im Bereich von 200 bis 375°C und Reaktionszeiten im Bereich von 10 bis 90, vorzugsweise von 10 bis 30 min. Als Lösungsmittel verwendet man in der Regel Wasser, wobei der 6-Aminocapronitril-Gehalt, bezogen auf das Wasser, im allgemeinen im Bereich von unter 30, vorzugsweise von 10 bis 25 Gew.-% gewählt wird.

Bei der Umsetzung in der Flüssigphase in Gegenwart eines Katalysators wählt man üblicherweise eine Temperatur im Bereich von 50 bis 330°C, eine Wassermenge im Bereich von 1,3 bis 50, bevorzugt von 1,3 bis 30 mol pro mol 6-Aminocapronitril und eine Reaktionszeit im Bereich von 10 min bis mehreren Stunden. Bei Verwendung eines organischen Lösungsmittels, insbesondere eines Alkohols, wählt man im allgemeinen eine Wassermenge im Bereich von 1,3 bis 5 mol pro mol 6-Aminocapronitril.

Üblicherweise arbeitet man den bei der Cyclisierung erhaltenen Reaktionsaustrag zunächst destillativ auf, wobei Ammoniak, Wasser und gegebenenfalls organisches Lösungsmittel abgetrennt werden. Der im Sumpf vorhandene Katalysator, falls eingesetzt, wird in der Regel vom Caprolactam nach einer der üblichen Methoden abgetrennt und in den Cyclisierungsreaktor zurückgeführt. Das Rohcaprolactam wird im allgemeinen durch an sich bekannte Reinigungsoperationen wie Destillation in Reinlactam überführt, das anschließend zur Polymerisation zu Polycaprolactam zur Verfügung steht.

In einer bevorzugten Ausführungsform setzt man 6-Aminocapronitril mit Wasser in flüssiger Phase unter Verwendung heterogener Katalysatoren um.

Die Umsetzung führt man in flüssiger Phase bei Temperaturen von im allgemeinen 140 bis 320°C, vorzugsweise 160 bis 280°C, durch; der Druck liegt im allgemeinen im Bereich von 100 kPa bis 25 Mpa, vorzugsweise von 500 kPa bis 15 MPa, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol 6-Aminocapronsäurenitril werden im allgemeinen mindestens 0,01 mol, vorzugsweise 0,1 bis 20 und insbesondere 1 bis 5 mol Wasser eingesetzt.

Vorteilhaft wird das 6-Aminocapronsäurenitril in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Wasser/Lösungsmittel-Gemischen eingesetzt. Als Lösungsmittel seien beispielhaft Alkanole wie Methanol, Ethanol, n- und i-Propanol, n-, sek.-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1 bis 75/25 bis 99, vorzugsweise 1 bis 50/50 bis 99 haben sich in einigen Fällen als besonders vorteilhaft herausgestellt.

Es ist prinzipiell genauso möglich, das 6-Aminocapronsäurenitril als Reaktand und gleichzeitig Lösungsmittel anzuwenden.

Als heterogene Katalysatoren können beispielsweise verwendet werden: saure, basische oder amphotere Oxide der Elemente der zweiten, dritten oder vierten Hauptgruppe des Periodensystems, wie Calciumoxid, Magnesiumoxid, Boroxid, Aluminiumoxid, Zinnoxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titandioxid, amorph, als Anatas und/oder Rutil, Zirkondioxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:

Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische Ionenaustauscher wie beispielsweise Naphion® als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann z. B. Titandioxid als Titandioxid-Strang oder als auf einen Träger in dünner Schicht aufgebrachtes Titandioxid eingesetzt werden. Zum Aufbringen von Titandioxid auf einen Träger wie Siliciumdioxid, Aluminiumoxid oder Zirkondioxid sind alle in der Literatur beschriebenen Methoden verwendbar. So kann eine dünne Titandioxid-Schicht durch Hydrolyse von Ti-Organylen wie Ti-Isopropylat oder Ti-Butylat, oder durch Hydrolyse von TiCl₄ der anderen anorganischen Ti-haltigen Verbindungen aufgebracht werden. Auch Titandioxid-haltige Sole sind verwendbar.

Weitere geeignete Verbindungen sind Zirkonylchlorid, Aluminiumnitrat und Cemitrat.

Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

In einer weiteren bevorzugten Ausführungsform cyclisiert man 6-Aminocapronsäurenitril in Flüssigphase mit Wasser bei erhöhter Temperatur ohne Katalysator, indem man eine waßrige Lösung von 6-Aminocapronsäurenitril in flüssiger Phase ohne Zusatz eines Katalysators in einem Reaktor erhitzt unter Erhalt einer Mischung I, bestehend aus im wesentlichen Wasser, Caprolactam und einer hochsiedenden Fraktion ("Hochsieder"). In dieser bevorzugten Ausführungsform setzt man Wasser bevorzugt im Überschuß ein, besonders bevorzugt verwendet man je mol 6-Aminocapronsäurenitril 10 bis 150, insbesondere 20 bis 100 mol Wasser unter Erhalt einer wäßrigen Lösung von 6-Aminocapronsäurenitril.

In einer weiteren bevorzugten Ausführungsform verwendet man üblicherweise 5 bis 25 mol Wasser je mol 6-Aminocapronsäurenitril und kann die Lösung im allgemeinen durch Zusatz eines organischen Lösungsmittels auf 5 bis 25 Gew.-% 6-Aminocapronsäurenitril weiter verdünnen.

Als geeignete Lösungsmittel seien beispielsweise genannt:

C₁-C₄-Alkanole wie Methanol, Ethanol, n-, i-Propanol, Butanole wie n-Butanol, Isobutanol, tert. Butanol und sek. Butanol, Glykole wie Ethylenglykol, Diethylenglykol, Triethylenglykol, Tetraethylenglykol, Ether wie Methyl-tert.-butylether, Diethylenglykoldiethylether, C₆-C₁₀-Alkane wie n-Hexan, n-Heptan, n-Octan, n-Nonan, n-Decan sowie Cyclohexan, Benzol, Toluol, Xylol, Lactame wie Pyrrolidon, Caprolactam oder N-C₁-C₄-Alkyl-Lactame wie N-Methylpyrrolidon, N-Methylcaprolactam oder N-Ethylcaprolactam.

In einer weiteren Ausführungsform kann man dem Reaktionsgemisch von 0 bis 5, bevorzugt von 0,1 bis 2 Gew.-% Ammoniak, Wasserstoff oder Stickstoff zusetzen.

Bevorzugt führt man die Reaktion bei einer Temperatur im Bereich von 200 bis 370, vorzugsweise von 220 bis 350°C, besonders bevorzugt von 240 bis 320°C durch.

Üblicherweise führt man die Reaktion unter Druck durch, wobei man den Druck in der Regel im Bereich von 0,1 bis 50, bevorzugt von 5 bis 25 MPa so wählt, daß das Reaktionsgemisch bevorzugt in flüssiger Phase vorliegt.

Die Reaktionsdauer hängt im wesentlichen von den gewählten Verfahrensparametern ab und liegt beim kontinuierlich durchgeführten Verfahren im allgemeinen im Bereich von 10 bis 180, bevorzugt von 20 bis 90 min. Bei kürzeren Reaktionszeiten sinkt in der Regel der Umsatz, bei längeren Reaktionszeiten bilden sich im allgemeinen störende Oligomere.

Die Cyclisierung führt man bevorzugt kontinuierlich, vorzugsweise in einem Rohrreaktor, in Rührkesseln oder Kombinationen davon durch.

Die Cyclisierung kann man auch diskontinuierlich durchführen.
Die Reaktionsdauer liegt dann üblicherweise im Bereich von 30 bis 180 min.

Der Austrag ist in der Regel eine Mischung, bestehend im wesentlichen aus 50 bis 98, vorzugsweise von 80 bis 95 Gew.-% Wasser und Lösungsmittel und von 2 bis 50, vorzugsweise von 5 bis 20 Gew.-% einer Mischung, bestehend im wesentlichen aus 0 bis 10 Gew.-% leichtsiedenden Anteilen, insbesondere Aminocapronitril und der entsprechende Aminocapronsäureester, bezogen auf die Caprolactam-haltige Mischung, aus von 50 bis 95, vorzugsweise von 65 bis 90 Gew.-% Caprolactam und von 5 bis 50, vorzugsweise von 10 bis 35 Gew.-% einer hochsiedenden Fraktion ("Hochsieder").

Erfindungsgemäß trennt man aus dem Reaktionsaustrag der Cyclisierung (Reaktionsaustrag I) in Stufe a) Caprolactam sowie Hochsieder durch Destillation, bevorzugt durch fraktionierte Destillation, ab.

In einer bevorzugten Ausführungsform entfernt man vor der Destillation von Caprolactam und den Hochsiedern Ammoniak und gewünschtenfalls Wasser sowie gegebenenfalls vorhandenes Lösungsmittel und/oder nicht umgesetztes 6-Aminocapronitril nach an sich bekannten Methoden wie sie beispielsweise in den US 2,301,964, DE-A 4,339,648, DE-A 4,422,610, EP-A 659,741, US 4,628,085 oder US 4,625,023 beschrieben sind. In einer weiteren bevorzugten Ausführungsform führt man Wasser, Lösungsmittel, so vorhanden, und 6-Aminocapronitril in die Cyclisierungsstufe zurück. Ammoniak wird im allgemeinen ausgeschleust.

Die in der Hochsiederspaltung (Stufe b)) eingesetzten Hochsieder können noch monomeres Caprolactam, z. B. 0,1 bis 50 Gew.-%, insbesondere 10 bis 40 Gew.-%, bezogen auf das Hochsiedergemisch, enthalten.

Für die Behandlung (Spaltung) der Hochsieder setzt man erfindungsgemäß als Säure Phosphorsäure und/oder Polyphosphorsäure ein. Die Phosphorsäure kann als wasserfreie oder wasserhaltige Phosphorsäure eingesetzt werden. Vorzugsweise verwendet man käufliche, 85 gew.-%ige wasserhaltige Phosphorsäure.

Die Menge an Säure (bei Phosphorsäure als 100%ige Säure berechnet) beträgt erfindungsgemäß 0,01 bis 10 Gew.-%, bevorzugt 0,02 bis 2 Gew.-%, besonders bevorzugt 0,03 bis 0,1 Gew.-%, bezogen auf die eingesetzten Hochsieder.

Die Hochsieder-Spaltung wird erfindungsgemäß bei Temperaturen von 200°C bis 350°C, bevorzugt 200°C bis 330°C, besonders bevorzugt 220°C bis 320°C durchgeführt.

Der Druck bei der Spaltung liegt üblicherweise im Bereich von 10 kPa bis 1 MPa, bevorzugt 50 kPa bis 500 kPa, besonders bevorzugt 80 kPa bis 200 kPa.

In einer bevorzugten Ausführungsform kann man die Hochsieder mit 85 gew.-%iger Phosphorsäure (0,075 Gew.-% Wasser, bezogen auf das Hochsiedergemisch) ohne zusätzliche Wasserdampfzufuhr zu Caprolactam spalten.

In einer weiteren bevorzugten Ausführungsform leitet man überhitzten Wasserdampf in das Hochsiedergemisch ein und trennt das gebildete monomere Caprolactam zusammen mit Wasserdampf von Phosphorsäure und verbleibenden Hochsiedern destillativ ab.

Die Temperatur des überhitzten Wasserdampfs wählt man im allgemeinen im Bereich von 180 bis 400°C, insbesondere 200 bis 350°C. Die in Form von Wasserdampf eingebrachte Wassermenge beträgt üblicherweise 0,05 g bis 20 g Wasser, bevorzugt 0,1 g bis 10 g, besonders bevorzugt 0,5 g bis 5 g pro g Hochsiedergemisch.

Die Verweilzeiten wählt man in Abhängigkeit von Temperatur, Druck, Säure- und Wassermenge üblicherweise im Bereich von 0,1 bis 7 Stunden, bevorzugt einer bis 5 Stunden.

Des weiteren kann man die Behandlung der Hochsieder mit Säure, insbesondere Phosphorsäure und Polyphosphorsäure, diskontinuierlich oder kontinuierlich durchführen.

Das durch Spaltung der Hochsiedergemische gebildete Caprolactam, das als wäßrige Lösung anfällt, kann noch geringe Mengen 6-Aminocapronitril enthalten.

Bei diskontinuierlicher Durchführung kann man z. B. so vorgehen, daß man ein Gemisch aus Hochsiedern und Säure, insbesondere Phosphorsäure, auf die gewünschte Reaktionstemperatur erhitzt, überhitzten Wasserdampf einträgt und das entstehende Caprolactam und gegebenenfalls 6-Aminocapronitril und Wasser über eine auf den Reaktor aufgesetzte Kolonne abdestilliert und als Sumpfprodukt ein Gemisch aus nicht umgesetzten Hochsiedern und eingesetzter Säure erhält. Das Sumpfprodukt kann nach einer bevorzugten Ausführungsform mehrfach wiederverwendet werden. Hierzu versetzt man es erneut mit Hochsiedern und wiederholt die Behandlung mit Wasserdampf.

Bei kontinuierlicher Durchführung werden in der Regel Hochsieder, Phosphorsäure und/oder Polyphosphorsäure und überhitzter Wasserdampf in einen Reaktor eingespeist. Man kann das Reaktionsgemisch nach Einhalten der Verweilzeit in Caprolactam und gegebenenfalls 6-Aminocapronitril und Wasser als Kopfprodukt und ein Gemisch aus Hochsiedern und Phosphorsäure und/oder Polyphosphorsäure als Sumpf produkt auftrennen. Dieses Sumpfprodukt kann, vorzugsweise, zurückgeführt werden.

Erfindungsgemäß trennt man das in Stufe b) gebildete Caprolactam und gegebenenfalls 6-Aminocapronitril aus dem Reaktionsaustrag der Stufe b) (Reaktionsaustrag II) von nicht umgesetzten Hochsiedern und eingesetzter Säure ab, wobei das abgetrennte Caprolactam - je nach gewünschtem Trennaufwand - noch Wasser und gewünschtenfalls 6-Aminocapronitril enthalten kann.

Das in Stufe c) abgetrennte Caprolactam kann man nach üblichen Methoden weiteren Reinigungsschritten unterwerfen.

In einer bevorzugten Ausführungsform schleust man das in Stufe c) erhaltenen Caprolactam in die Aufarbeitungsstufe des bei der Cyclisierung von 6-Aminocapronitril mit Wasser anfallenden Rohcaprolactams ein und/oder vereinigt es mit dem Caprolactam, das aus Stufe a) erhalten wird.

Die Herstellung von spezifikationsgerechtem Reinlactam aus dem bei der 6-Aminocapronitril-Cyclisierung erhaltenen Rohcaprolactam und dem zugesetzten Spaltcaprolactam kann z. B. nach dem in der US 5,496,941 beschriebenen Verfahren erfolgen. Führt man die dort ausgeführte Reaktionssequenz aus katalytischer Hydrierung, saurer und alkalischer Destillation durch, so wird ein Reincaprolactam erhalten, das die Spezifikation im Hinblick auf die für "Beckmann-Caprolactam" typische Kennzahlen wie Permanganattitrationszahl (PTZ), Permanganatabsorptionszahl (PAZ), UV-Kennzahl, freie und flüchtige Basen erfüllt.

Es war überraschend, daß man aus den Hochsiedergemischen der Formel I Caprolactam und 6-Aminocapronitril mit Ausbeuten von über 90 % gewinnen kann. Da die Zahl der 6-Aminocapronsäure-Einheiten in den Hochsiedern im Mittel unter fünf liegt, müssen auch die unterschiedlichen Endglieder (R = -CO-NH₂; -CN, -COOR) in hohem Maße zur Caprolactam-Bildung beigetragen haben. Daß dies unter den Bedingungen der Phosphorsäurespaltung möglich ist, war nicht vorherzusehen.

Weiterhin war überraschend, daß man mit sehr geringen Phosphorsäure- und/oder Polyphosphorsäure-Mengen und relativ geringen Wassermengen höhere Caprolactam-Ausbeuten als nach dem Stand der Technik erzielt.

Schließlich war nicht vorherzusehen, daß das aus Gemischen aus Cyclisierungs- und Spaltlactam nach Reinigung (Hydrierung, saure Destillation, alkalische Destillation) erhaltene Reinlactam den gewünschten Spezifikationsanforderungen entsprechen würde.

### Beispiele

### Beispiel 1

### Caprolactam durch Cyclisierung von 6-Aminocapronitril

In einem auf 225°C geheizten Rohrreaktor von 20 ml Inhalt (Durchmesser 6 mm, Länge 710 mm), der mit Titandioxid (Anatas) in Form von 1,5 mm Strängen gefüllt war, wurden bei 100 bar 70 g/h einer Lösung aus 10 Gew.-% 6-Aminocapronitril, 3,2 Gew.-% Wasser und Ethanol (Rest) umgesetzt.

Die quantitative gaschromatographische Auswertung des Reaktionsaustrags ergab folgende Ausbeuten: 90 % Caprolactam, 4 % 6-Aminocapronsäureethylester sowie 2 % 6-Aminocapronitril.

Ein während 350 Stunden gesammelter Produktstrom wurde von Ammoniak, Ethanol und Wasser befreit und das so erhaltene Rohlactam destilliert. Dabei fielen 102 g leichtsiedende Anteile ("Leichtsieder") und 226 g hochsiedende Anteile ("Hochsieder") mit einem Siedepunkt von größer als 190°C bei einem Druck von 1 mbar sowie 2140 g Caprolactam an. Der Leichtsieceranteil setzte sich im wesentlichen aus 6-Aminocapronsäureechylester und unumgesetztem 6-Aminocapronitril, der Hochsiederanteil aus Oligomeren zusammen.

In den folgenden Beispielen enthalten die für die Spaltversuche nach diesem Beispiel hergestellte Hochsieder teilweise noch monomeres Caprolactam.

### Beispiel 2

### Caprolactam durch Spaltung der Hochsieder mit Phosphorsäure (ohne Wasserdampf)

In einem 500 ml Dreihalskolben mit aufgesetzter Kolonne wurde ein Gemisch aus 250 g der nach Beispiel 1 hergestellten Hochsieder, welche in einer weiteren Destillation vom Rest-Caprolactam befreit wurde, und 1,25 g 85 gew.-%iger Phosphorsäure erhitzt. Bei einer Sumpftemperatur von 350°C destillierten bei 250 bis 270°C/1013 mbar 166 g eines Produktgemisches über, das laut gaschromatographischer Analyse 151 g Caprolactam (60 %, bezogen auf eingesetzten Hochsieder) enthielt.

### Beispiel 3

### Caprolactam durch Spaltung der Hochsieder mit Phosphorsäure (mit Wasserdampf)

In einem 500 ml-Dreihalskolben mit aufgesetzter Kolonne wurde ein Gemisch aus 250 g der nach Beispiel 1 hergestellten Hochsieder (Gehalt an monomerem Caprolactam: 36 %) und 1,25 g 85 gew.-%iger Phosphorsäure auf 350°C erhitzt. Während eines Zeitraums von 70 Minuten wurden bei dieser Temperatur und bei Normaldruck 500 g/h auf 350°C überhitzter Wasserdampf eingeleitet. Über die Kolonne wurden nach Kondensation 724 g einer wäßrigen Lösung erhalten, die laut gaschromatographischer Analyse 30,2 % Caprolactam und 0,6 % 6-Aminocapronitril enthielt. Im Reaktionskolben blieben 10 g eines Gemisches aus Hochsiedern und Katalysator zurück.

Dieses Beispiel zeigt, daß die eingesetzten Hochsieder zu 80 % in Caprolactam (bezogen auf eingesetzten Hochsieder) umgewandelt werden können. Außerdem wird deutlich, daß nur 6 % Hochsieder + Phosphorsäure (bezogen auf die eingesetzte Hochsieder) entsorgt werden müssen.

### Beispiel 4

### Caprolactam durch Spaltung der Hochsieder mit Polyphosphorsäure (mit Wasserdampf)

In einem 500 ml-Dreihalskolben mit aufgesetzter Kolonne wurde ein Gemisch aus 250 g des nach Beispiel 1 hergestellten Hochsieders (Gehalt an monomerem Caprolactam: 36 %) und 1,25 g handelsübliche Polyphosphorsäure (Dichte = 2,8 g/ml) auf 250°C erhitzt. Während eines Zeitraums von 3 Stunden wurden bei dieser Temperatur und bei Normaldruck 250 g/h auf 300°C überhitzter Wasserdampf eingeleitet. Über die Kolonne wurden nach Kondensation 1075 g einer wäßrigen Lösung erhalten, die laut gaschromatographischer Analyse 21,8 % Caprolactam und 0,2 % 6-Aminocapronitril enthielt. Im Reaktionskolben blieben 6 g eines Hochsieder-Katalysator-Gemisches zurück.

Derversuch zeigt, daß die eingesetzten Hochsiederzu 90 % in Caprolactam umgewandelt werden. Außerdem wird deutlich, daß nur 4 % Hochsieder (bezogen auf die eingesetzte Hochsiedermenge) entsorgt werden müssen.

### Beispiel 5

Der in Beispiel 4 nach der Hochsiederspaltung mit Polyphosphorsäure erhaltene Rückstand (6 g) wurde erneut mit 250 g eines Hochsieder-Gemisches (Gehalt an monomerem Caprolactam 90 g) versetzt. Die Caprolactam-Gewinnung erfolgte wie im Beispiel 4 beschrieben. Die Caprolactam-Ausbeute betrug nach Abzug von schon enthaltenem monomerem Caprolactam 89 %. Im Reaktionskolben blieben 11 g Hochsieder zurück.

### Beispiel 6

### Caprolactam und 6-Aminocapronitril durch Spaltung des Hochsieders Caprolactim-(6-amino-capronsäurenitril) (1) mit Polyphosphorsäure (mit Wasserdampf)

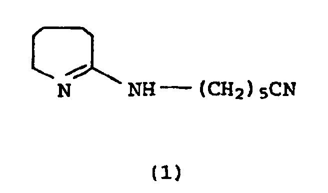

(1) entsteht nach WO 9636601-11 durch Erhitzen von 6-Aminocapronitril.

In einem 500 ml Glaskolben wurden 250 g Caprolactim(6-aminocapronsäurenitril) und 5 g Polyphosphorsäure vorgelegt. Das Gemisch wurde auf 250°C erhitzt und auf 270°C überhitzter Wasserdampf mit 125 g/h eingeleitet. Der das Reaktionsgemisch verlassende Gasstrom wurde aufgefangen und verflüssigt. Man erhielt in 6 h 970 g Reaktionsaustrag, in dem 181 g Caprolactam,. weiterhin 5,1 g nicht umgesetztes Edukt und 71,9 g 6-Aminocapronitril gelöst waren, die wiederum zum Caprolactam umgesetzt werden können. Die Caprolactamausbeute beträgt 66 %, die Wertproduktselektivität 93 %.

### Beispiel 7

Gewinnung von spezifikationsgerechtem Caprolactam durch Reinigung von Gemischen aus Cyclisierungs- und Spaltlactam.

Die Caprolactam-Reinigung wurde analog zu U.S. 5.496.941 (DE 195 000 41), Beispiel 1 durchgeführt.
a) Herstellung des Cyclisierungscaprolactams
   Das Cyclisierungslactam wurde nach Beispiel 1 durch Cyclisierung von 6-Aminocapronitril an TiO₂ hergestellt. Der Cyclisierungsaustrag wurde destillativ erst von Ammoniak, Ethanol und Wasser befreit. Das so erhaltene Rohlactam wurde dann destillativ von Leichtsiedern und Hochsiedern befreit.
b) Herstellung des Spaltcaprolactams
   Nach Beispiel 1 gewonnener, Caprolactam-freier Hochsieder wurde nach Beispiel 4 in Gegenwart von Polyphosphorsäure und Wasserdampf zu Caprolactam gespalten. Nach der destillativen Abtrennung des Wassers wurde das Rohcaprolactam destillativ von Hoch- und Leichtsiedern befreit.
c) Reinigung

1000 g eines Gemisches aus 900 g nach a) und 100 g nach b) gewonnenem Rohcaprolactam wurden in 250 g Wasser gelöst. Die wäßrige Lösung wurde in einem Autoklaven mit 3,5 g 5 gew.-%igem Palladium auf Aktivkohle versetzt und unter Rühren vier Stunden lang bei 80°C/5 bar hydriert.

Nach dem Abkühlen und Entspannen des Autoklaven wurde der Katalysator abfiltriert. Das Filtrat wurde bei 50°C und Normaldruck in Rieselfahrweise innerhalb von 0,6 Stunden über 1 l eines starksauren Ionentauschers (Amberlite® IR 120, H-Form) geleitet.

Der Ionentauscher-Austrag wurde mit 4 g 25 %iger wäßriger Natronlauge versetzt. In eine Destillationskolonnne mit 2 theoretischen Böden wurde das Wasser bei einem Kopfdruck von 48 mbar und einer Sumpftemperatur von 134°C abdestilliert.

Aus dem Sumpfprodukt der ersten Kolonne wurden in einer zweiten Kolonne mit 15 theoretischen Böden die Leichtsieder bei einem Kopfdruck von 4 mbar und einer Sumpftemperatur von 143°C abdestilliert.

Das Sumpfprodukt der zweiten Kolonne wurde in einer dritten Kolonne mit 15 theoretischen Böden destilliert. Bei einem Kopfdruck von 5 mbar und einer Sumpftemperatur von 150°C wurden insgesamt 982 g Caprolactam über Kopf destilliert (98 % bezogen auf eingesetztes Rohcaprolactam). Das so erhaltene Reinlactam besaß folgende Kennzahlen (Analysenvorschriften siehe DE 195 000 41):

| | Beispiel 7 | DE 195 000 41, Beispiel 1 | BASF-Spezifikation |
|---|---|---|---|
| PAZ: | 2,5 | 1,5 | 4 |
| PTZ: | 2,5 | 1,2 | - |
| freie Basen: | < 0,05 meq/kg | < 0,05 meq/kg | < 0,1 meq/kg |
| flüchtige Basen: | < 0,5 meq/kg | < 0,5 meq/kg | < 0,5 meq/kg |
| UV: | 2,5 | 2,5 | - |

Beispiel 7 zeigt, daß auch aus Caprolactam-Gemischen, die durch Spaltung von Hochsiedern mit Phosphorsäure und Wasser erzeugtes Caprolactam enthalten, spezifikationsgerechtes Caprolactam erhalten werden kann.

## Patentansprüche

1. Verfahren zur Herstellung von Caprolactam und höher als Caprolactam siedenden Komponenten ("Hochsieder") der Formel I 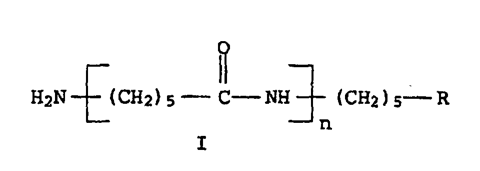 mit
R: Carbonsäure-, Carbonsäureamid-, und Nitrilgruppe und, bei Verwendung von Alkoholen als Lösungsmittel bei der Cyclisierung von 6-Aminocapronitril, zusätzlich und/ oder Estergruppe
n: ganze Zahl von 1 bis 50, wobei der Durchschnittswert von n über alle Verbindungen I kleiner als 5 ist.
durch Cyclisierung von 6-Aminocapronitril in Gegenwart von Wasser bei erhöhter Temperatur und gewünschtenfalls eines Katalysators sowie eines Lösungsmittels, **dadurch gekennzeichnet, daß** man
a) aus einem Reaktionsaustrag ("Reaktionsaustrag I") der Cyclisierung Caprolactam und Hochsieder abtrennt,
b) die Hochsieder aus Stufe a) mit 0,01 bis 10 Gew.-% Phosphorsäure und/oder Polyphosphorsäure, bezogen auf die eingesetzten Hochsieder, bei einer Temperatur im Bereich von 200 bis 350°C behandelt unter Erhalt eines Reaktionsaustrages II und
c) aus dem Reaktionsaustrag II der Stufe B) gebildetes Caprolactam und gegebenenfalls 6-Aminocapronitril von nicht umgesetzten Hochsiedern und eingesetzter Säure abtrennt.

2. Verfahren gemäß Anspruch 1, **dadurch gekennzeichnet, daß** man in Stufe b) 0,05 g bis 20 g Wasser pro g Hochsieder in Form von überhitztem Wasserdampf in das Reaktionsgemisch einbringt.

3. Verfahren gemäß Anspruch 1 oder 2, **dadurch gekennzeichnet, daß** man vor der Destillation von Caprolactam und Hochsiedern in Stufe a) Ammoniak und gewünschtenfalls Wasser sowie gegebenenfalls vorhandenes Lösungsmittel destillativ abtrennt.

4. Verfahren gemäß den Ansprüchen 1 bis 3, **dadurch gekennzeichnet, daß** man das in Stufe c) abgetrennte Caprolactam gegebenenfalls nach Abtrennung von 6-Aminocapronitril, mit dem Caprolactam aus Stufe a) vereinigt und gewünschtenfalls das vereinigte Caprolactam in üblicher Weise zu spezifikationsgerechtem Caprolactam aufarbeitet.

## Claims

1. A process for preparing caprolactam and components boiling higher than caprolactam ("high boilers") of the formula I 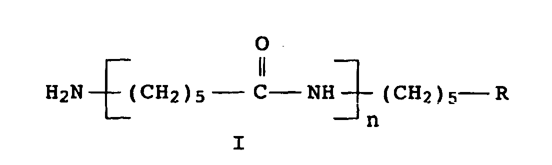 where
R is carboxyl, carbamoyl and nitrile or, when alcohols are used as solvents in the cyclization of 6-aminocapronitrile, additionally and/or an ester group
n is an integer from 1 to 50, but the average value of n over all compounds I is less than 5,
by cyclization of 6-aminocapronitrile in the presence of water at elevated temperature and in the presence or absence of a catalyst and a solvent, which comprises
a) removing caprolactam and high boilers from a reaction effluent ("reaction effluent I") of the cyclization,
b) treating the high boilers of stage a) with from 0.01 to 10% by weight of phosphoric acid and/or polyphosphoric acid, based on the high boilers used, at from 200 to 350°C to obtain a reaction effluent II, and
c) separating caprolactam formed from said reaction effluent II of stage b) and if appropriate 6-aminocapronitrile from unconverted high boilers and acid used.

2. The process according to claim 1 wherein from 0.05 g to 20 g of water per g of high boilers is introduced into the reaction mixture of stage b) in the form of superheated steam.

3. The process according to claim 1 or 2 wherein ammonia and, if desired, water, and also if appropriate solvent present are removed by distillation before the distillation of caprolactam and high boilers in stage a).

4. The process according to any of claims 1 to 3 wherein the caprolactam separated off in stage c) is, if appropriate after removal of 6-aminocapronitrile, combined with the caprolactam of stage a) and, if desired, the combined caprolactam is worked up in a conventional manner to on-spec caprolactam.

## Revendications

1. Procédé de préparation de caprolactame et de composants à point d'ébullition supérieur à celui du caprolactame ("composants lourds") de la formule I 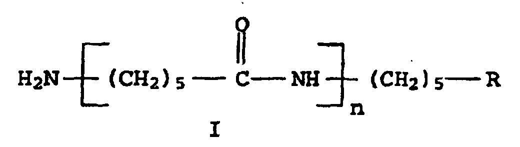 avec
R : radicaux d'acide carboxylique, amide d'acide carboxylique et nitrile, lorsque l'on utilise des alcools en tant que solvants lors de la cyclisation du 6-aminocapronitrile, additionnellement et/ou des radicaux ester
n : nombre entier de 1 à 50, la valeur moyenne de n pour tous les composés I étant inférieur à 5,
par cyclisation de 6-aminocapronitrile en présence d'eau à haute température et éventuellement d'un catalyseur ainsi qu'un solvant,
**caractérisé en ce que**
a) on sépare d'un produit de réaction ("produit de réaction I") de cyclisation le caprolactame et les produits lourds,
b) on traite les produits lourds de l'étape a) par 0,01 à 10% en poids d'acide phosphorique et/ou d'acide polyphosphorique, par rapport aux produits lourds individuels, à une température de l'ordre de 200 à 350°C, en obtenant un produit de réaction II, et
c) on sépare du produit de réaction II de l'étape B) le caprolactame formé et éventuellement le 6-aminocapronitrile des produits lourds non convertis et des acides mis en oeuvre.

2. Procédé selon la revendication 1, **caractérisé en ce que** l'on introduit dans l'étape b), dans le mélange réactionnel, de 0,05 à 20 g d'eau par g de produit lourds, sous la forme de vapeur d'eau surchauffée.

3. Procédé selon la revendication 1 ou 2, **caractérisé en ce qu'**avant la distillation du caprolactame et des produits lourds à l'étape a), on sépare par distillation de l'ammoniac et éventuellement de l'eau ainsi que les solvants éventuellement présents.

4. Procédé selon les revendications 1 à 3, **caractérisé en ce que** l'on réunit le caprolactame isolé à l'étape c) après séparation du 6-aminocapronitrile, au caprolactame de l'étape a), et que le caprolactame éventuellement rassemblé est retraité de la manière usuelle pour donner du caprolactame conforme aux spécifications.
